# EUROPÄISCHE PATENTSCHRIFT

(11) **EP 2 148 200 B1**
(45) Veröffentlichungstag und Bekanntmachung des Hinweises auf die Patenterteilung: **19.01.2011**
(21) Anmeldenummer: 09176202.1
(22) Anmeldetag: 21.08.2007
(51) Int. Cl.: G01N 33/487

(54) **Diagnostische Bandkassette, insbesondere für Blutzuckertests**
Diagnostic tape cassette, in particular for blood sugar tests
Cassette à bande diagnostique, en particulier pour mesurer le taux de sucre sanguin

(30) Priorität: 22.08.2006 EP 06017404
(43) Veröffentlichungstag der Anmeldung: 27.01.2010
(62) Teilanmeldung aus: 07802742.2
(73) Patentinhaber: F. Hoffmann-La Roche AG, 4070 Basel (CH); Roche Diagnostics GmbH, 68305 Mannheim (DE)
(72) Erfinder: Sacherer, Klaus-Dieter, 67281, Kirchheim (DE)
(74) Vertreter: Pfiz, Thomas

(56) Entgegenhaltungen:
- US-A- 4 115 067
- US-A- 5 171 397
- US-A1- 2002 079 345
- US-A1- 2002 188 224

## Beschreibung

Die Erfindung betrifft eine diagnostische Bandkassette, insbesondere für Blutzuckertests, mit einem analytischen Testband, einer Vorratsspule zum Abspulen von unverbrauchtem Testband sowie einer Aufwickelspule zum Aufwickeln von verbrauchtem Testband, einem Gehäuse für die Spulen und einer Drehsicherung für die Aufwickelspule zumindest gegen ungewolltes Abwickeln von Testband.

Für die Selbstdiagnose von Diabetikern werden bislang in der Praxis einzelne Teststreifen eingesetzt, die nach der Applikation einer geringen Probenmenge photometrisch untersucht werden, um den Glucosegehalt in der Probe (Blut bzw. Gewebeflüssigkeit) möglichst genau und zuverlässig zu bestimmen. Zur Verbesserung der Anwenderfreundlichkeit wurde bereits vorgeschlagen, eine Vielzahl von Tests auf einem Testband in Form einer Bandkassette bereitzustellen. Solche Bandkassetten sollen sich als Einwegteil in kompakte Handgeräte einsetzen lassen, um alle erforderlichen Untersuchungsschritte automatisch und schnell ausführen zu können.

In diesem Zusammenhang ist in der zum Prioritätszeitpunkt dieser Anmeldung noch unveröffentlichten DE 10 2005 013 685 der Anmelderin eine Rücklaufsicherung gegen Bandabwickeln offenbart, um ein ungewolltes Ausspulen von gebrauchtem und mit Blut kontaminiertem Band zu verhindern. Die Sicherung soll dort in Form eines Freilaufgesperres ein Aufwickeln von Testband erlauben, während das Zurückdrehen in Gegenlaufrichtung gesperrt werden soll. Hierbei ist zu beachten, dass diagnostische Bandkassetten als Verbrauchsteile ein Massenprodukt darstellen, welches möglichst einfach herstellbar sein soll und zuverlässig und mit hoher Benutzerfreundlichkeit arbeiten soll.

Weiterhin wird in der US 2002/188224 eine diagnostische Bandkassette mit einem Testband, einer Vorratsspule und einer Aufwickelspnle mit Rücklaufsperre offenbart.

Ausgehend hiervon liegt der Erfindung die Aufgabe zugrunde, die im Stand der Technik vorgeschlagenen Testbandsysteme weiter zu verbessern und bei einfacher Herstellbarkeit besondere Anwendungsvorteile zu erreichen.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Ein erstes Konzept der Erfindung geht von dem Gedanken aus, anstelle einer drehrichtungsgesteuerten Sperre einen selbsttätig schaltenden Mechanismus zu verwirklichen. Dementsprechend wird vorgeschlagen, dass die Drehsicherung jeweils eine an gegeneinander weisenden Flächen des Gehäuses und der Aufwickelspule angeordnete, durch eine Axialbewegung der Aufwickelspule in ihrer Spulenachse zwischen einer gegenseitigen Eingriffstellung und einer Freigabestellung schaltbare Sperrverzahnung aufweist. Auf diese Weise ist es möglich, eine Sperrwirkung situationsgerecht einzusetzen bzw. außer Kraft zu setzen. In der Eingriffstellung wird zuverlässig verhindert, dass gebrauchtes Testband durch Manipulation des Benutzers frei zugänglich wird. In der Freigabestellung werden störende Geräusche völlig vermieden, und es ist kein zusätzliches Antriebsdrehmoment erforderlich, was auch Vorteile im Hinblick auf die Energieversorgung beim Einsatz in Handgeräten bringt. Weiterhin arbeitet die vorgeschlagene Lösung unabhängig von dem Wickeldurchmesser des aufgewickelten Testbands, und die Drehsicherung kann mit wenigen Bauteilen ohne komplizierte Freilaufmechaniken hergestellt werden. Zugleich kann die Baugröße gering gehalten werden, so dass die Funktionsteile nicht wesentlich bestimmend für die Kassettengröße werden.

Bevorzugt stehen die Sperrverzahnungen bei geräteunabhängiger Handhabung der Bandkassette in der Eingriffstellung, und bei einem Geräteeinsatz der Bandkassette vorzugsweise durch Einführen einer geräteseitigen Antriebsspindel in die Aufwickelspule in der Freigabestellung.

Durch die axiale Bewegung der Sperrverzahnungen wird auch erreicht, dass die Aufwickelspule in der Freigabestellung in beide Drehrichtungen frei drehbar ist, und dass in der Eingriffstellung beide Drehrichtungen der Aufwickelspule gesperrt sind.

Eine konstruktiv besonders vorteilhafte Ausführung sieht vor, dass die Aufwickelspule eine an einer Stirnseite verzahnte Zahnscheibe als Sperrverzahnung aufweist, wobei die Zahnscheibe durch einen Bund eines mit dem Testband umwickelbaren zylinderförmigen Spulenkörpers gebildet sein kann.

Um eine selbsttätige Schaltfunktion zu realisieren, ist es von Vorteil, wenn die Aufwickelspule über eine Rückstellfeder in Richtung der Spulenachse an dem Gehäuse abgestützt ist. Hierbei ist es günstig, wenn die Rückstellfeder vorzugsweise als Blattfeder oder Spiralfeder an einer Wand des Gehäuses angeformt ist, und wenn zwischen der Wand und der Sperrverzahnung ein Ringraum für das Testband freigehalten ist.

Vorteilhafterweise besitzt das Gehäuse einen verzahnten Ringflansch als Sperrverzahnung.

Um eine ungewollte Sperrwirkung bei axialer Verkippung durch hohen Bandzug zu vermeiden, ist es von Vorteil, wenn die gehäuseseitige Sperrverzahnung an einem von einer letzten Umlenkstelle für das aufzuwickelnde Testband abgewandten Ringsegment ausgebildet ist, und wenn das verbleibende Ringsegment frei von einer Verzahnung ist.

Ein verbesserter Formschluss in Sperrrichtung lässt sich dadurch erreichen, dass die Zähne der Sperrverzahnungen unsymmetrische Zahnflanken aufweisen, wobei die beim Rückdrehen der Aufwickelspule in der Eingriffstellung gegeneinander anschlagenden Zahnflanken steiler sind.

Für eine einfache Herstellung ist es von Vorteil, wenn die Aufwickelspule in eine von einer körperlichen Drehachse freie Kammer des Gehäuses eingesetzt ist. Montageerleichterungen können sich auch dadurch ergeben, dass die Aufwickelspule in einer beliebigen Drehstellung auf die Sperrverzahnung des Gehäuses aufsetzbar ist.

Um insbesondere bei einem gesondert montierten Drehantrieb vorhandene Toleranzen ausgleichen zu können, ist es vorteilhaft, wenn die Aufwickelspule mit Querspiel bezüglich ihrer Spulenachse schwimmend in dem Gehäuse gelagert ist. Hierbei sollte gewährleistet sein, dass das Querspiel größer als 0,2 mm ist und vorzugsweise 0,3 bis 0,6 mm beträgt. Eine vorteilhafte Ausgestaltung sieht vor, dass die Aufwickelspule einen Ringfortsatz aufweist, welcher zumindest in der Eingriffstellung der Sperrverzahnungen mit Querspiel in eine Gehäuseöffnung eingreift.

Gemäß einem zweiten Erfindungskonzept weist die Drehsicherung ein an einer Gehäusefläche angebrachtes Reibelement auf, welches bei einer Axialbewegung der Aufwickelspule aus einer Freigabestellung in Reibschluss mit einer zugewandten Anlagefläche der Aufwickelspule gelangt. Dadurch kann auf baulich einfache Weise in jeder Drehstellung eine drehrichtungsunabhängige Sperrfunktion erreicht werden.

Vorteilhafterweise ist das Reibelement durch einen koaxial zu der Aufwickelspule angeordneten Reibring gebildet, aus einem Elastomermaterial, vorzugsweise aus TPE bestehen kann und vorzugsweise durch Spritzgießen an der Gehäusefläche angeformt ist.

Eine weitere Verbesserung wird dadurch erzielt, dass die Gehäusefläche durch einen eine Öffnung begrenzenden Ringflansch gebildet ist, und dass das Reibelement auf dem Ringflansch vorzugsweise im Bereich des Randes der Öffnung umlaufend angeordnet ist. Entsprechend ist es günstig, wenn die Anlagefläche durch einen Bund eines mit dem Testband umwickelbaren zylinderförmigen Spulenkörpers gebildet ist. Die Reibkraft ist damit unabhängig vom momentanen Durchmesser des Bandwickels auf dem Spulenkörper.

Um bei Nichtgebrauch der Kassette den Reibschluss selbsttätig sicherzustellen, ist es vorteilhaft, wenn die Aufwickelspule über eine Rückstellfeder in Richtung der Spulenachse an dem Gehäuse abgestützt ist, so dass der Reibschluss unter der Rückstellkraft der Rückstellfeder erfolgt.

Im Folgenden wird die Erfindung anhand der in der Zeichnung schematisch dargestellten Ausführungsbeispiele näher erläutert. Es zeigen:
- Fig. 1: eine Bandkassette für Blutzuckertests mit einer Abwickelsicherung für das Testband in einer perspektivischen Darstellung;
- Fig. 2: eine Aufwickelspule der Bandkassette mit einer Sperrverzahnung in vergrößerter perspektivischer Ansicht;
- Fig. 3: einen Ringflansch der Bandkassette mit einer Gegenverzahnung in der Draufsicht;
- Fig. 4 und 5: die Aufwickelspule und den Ringflansch in einer Eingriffstellung und einer Freigabestellung der Sperrverzahnung im Axialschnitt;
- Fig. 6: ein tragbares Messgerät mit einer Aufnahme für die Bandkassette in einer perspektivischen Darstellung;
- Fig. 7: eine Antriebsspindel des Geräts nach Fig. 6 in der Seitenansicht;
- Fig. 8 bis 10: eine weitere Ausführungsform der Bandkassette mit ei- ner Aufwickelspule gemäß Fig. 8 für einen Reibeingriff an einem gehäuseseitigen Reibring gemäß Fig. 9 oder 10 in perspektivi- scher Darstellung.

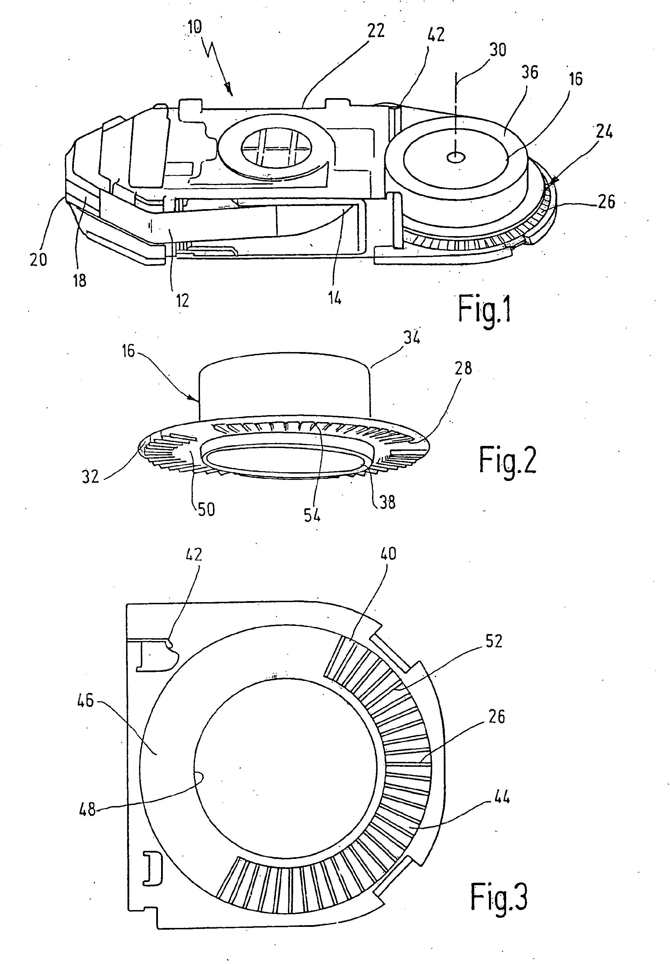

Die in Fig. 1 bei abgenommenem Deckel dargestellte Bandkassette ermöglicht die Durchführung einer Vielzahl von Glucoseuntersuchungen an vor Ort vom Patienten selbst gewonnenen Blutproben. Zu diesem Zweck umfasst die Bandkassette 10 ein analytisches Testband 12, das von einer Vorratsspule 14 abziehbar und auf eine Aufwickelspule 16 aufwickelbar ist, wobei ein Testfeld 18 an einer Applikationsspitze 20 umgelenkt wird, um eine vorderseitige Applikation von Körperflüssigkeit (Blut bzw. Gewebeflüssigkeit) und eine rückseitige reflektometrische Vermessung zu erlauben. Die abschnittsweise auf dem Testband 12 aufgebrachten Testfelder 18 enthalten Trockenchemikalien, die auf den Analyten (Glucose) in der applizierten Blutflüssigkeit ansprechen und bei rückseitiger Beleuchtung zu einer messbaren Veränderung der Lichtrückstrahlung führen.

Die das Testband 12 tragenden Spulen 14, 16 sind in ein Gehäuse 22 der Kassette 10 eingesetzt, in welchem bei aufgesetztem Deckel nur die Applikationsspitze 20 offen zugänglich ist. Um bei Manipulationen von außen ein ungewolltes Abwickeln von verbrauchtem und mit Blut kontaminiertem Testband zu verhindern, ist zwischen Aufwickelspule 16 und Gehäuse 22 ein Drehgesperre 24 vorgesehen. Dieses ist durch jeweils eine an dem Gehäuse 22 und der Aufwickelspule 16 ausgebildete Sperrverzahnung 26, 28 gebildet, welche durch eine Axialbewegung der Aufwickelspule längs der Spulenachse 30 zwischen einer gegenseitigen Eingriffbestellung und einer Freigabestellung schaltbar ist.

Wie am besten aus Fig. 2 ersichtlich, weist die Aufwickelspule 16 eine stirnseitig verzahnte Zahnscheibe 32 auf, welche die Sperrverzahnung 28 nach Art eines Kronenrades trägt. Die Zahnscheibe 32 ist durch einen Bund eines mit dem Testband 12 umwickelbaren hohlzylindrischen Spulenkörpers 34 gebildet, wobei die Sperrverzahnung 28 von dem Testbandwickel 36 (Fig. 1) abgewandt ist. An der verzahnten Seite besitzt die Aufwickelspule 16 einen Ringfortsatz 38 für eine schwimmende Zentrierung in der Gegenverzahnung 26.

Wie in Fig. 3 gezeigt, besitzt das Gehäuse 22 einen Ringflansch 40, welcher die gehäuseseitige Sperrverzahnung 26 trägt. Diese ist an einem von der in Bandtransportrichtung gesehen letzten Umlenkstelle 42 für das aufzuwickelnde Testband 12 abgewandten Ringsegment 44 ausgebildet, während das verbleibende Ringsegment 46 unverzahnt ist, so dass bei einer Verkippung aufgrund starker Bandzugbelastung der Aufwickelspule 16 ein ungewolltes Sperren vermieden wird. Der Ringflansch 40 begrenzt eine Gehäuseöffnung 48, in welcher der Ringfortsatz 38 der Aufwickelspule 16 in der Eingriffstellung mit Querspiel 49 gelagert ist. Dieses Querspiel 49 wird dadurch erreicht, dass die Gehäuseöffnung 48 einen um ca. 0,5 mm größeren Durchmesser als der Ringfortsatz 38 an seinem Umfang besitzt. Die Sperrverzahnungen 26, 28 erlauben aufgrund ihrer radialen Erstreckung eine entsprechende Querauslenkung der Aufwickelspule 16, wodurch Positioniertoleranzen ausgeglichen werden können.

Die Sperrverzahnungen 26, 28 sind somit an einander zugewandten Flächen 44, 50 ringförmig ausgebildet, so dass die Aufwickelspule 16 in einer beliebigen Drehstellung auf den Ringflansch 40 aufsetzbar ist. Um die Sperrwirkung gegen Rückdrehen zu verbessern, weisen die Zähne der Sperrverzahnungen 26, 28 unsymmetrische Zahnflanken auf, wobei die beim Rückdrehen der Aufwickelspule 16 in der Eingriffstellung gegeneinander anschlagenden Zahnflanken 52, 54 steiler als die jeweils gegenüber liegenden Zahnflanken sind.

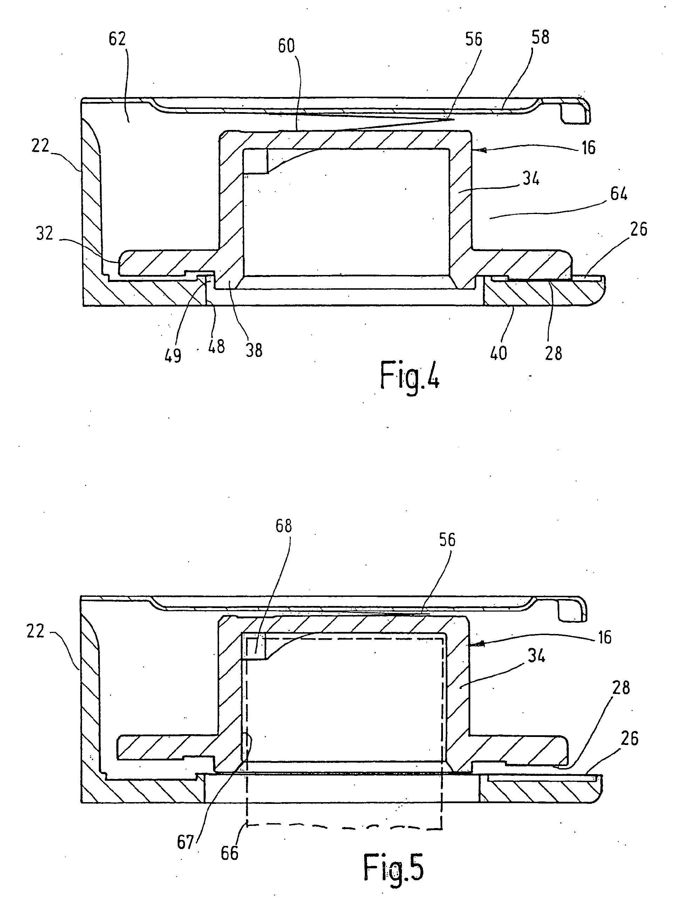

Die Schaltfunktion des Drehgesperres 24 ist am besten aus Fig. 4 und 5 ersichtlich. Bei Aufbewahrung oder geräteunabhängiger Handhabung der Bandkassette 10 stehen die Sperrverzahnungen 26, 28 in gegenseitigem Eingriff, so dass durch Formschluss beide Drehrichtungen gesperrt sind (Fig. 4). Hierfür sorgt eine Rückstellfeder 56, welche zwischen dem Deckel 58 des Gehäuses 22 und der geschlossenen Stirnfläche 60 des Spulenkörpers 34 eingespannt ist und diesen gegen den Ringflansch 40 drängt. Zweckmäßig ist die Rückstellfeder 56 als Spiralfeder an dem Deckel 58 angeformt. Die Aufwickelspule 16 lässt sich dann in die Gehäusekammer 62 einsetzen, wobei keine materielle Drehachse erforderlich ist und der Ringraum 64 zwischen dem Deckel 58 und dem Drehgesperre 24 zur Aufnahme des Testbands 12 freigehalten ist.

Bei Gebrauch der Bandkassette 10 greift eine in Fig. 5 nur schematisch gezeigte Antriebsspindel 66 eines geräteseitigen Antriebs in die zentrale Mitnahmebohrung 67 des Spulenkörper 34 axial ein und bewirkt dadurch unter Kompression der Feder 56 ein Ausheben in die Freigabestellung. Hierbei treten die Sperrverzahnungen 26, 28 außer Eingriff, und die Aufwickelspule 16 kann somit frei gedreht werden. Die Antriebsspindel 66 übernimmt dann ggf. die Sperrfunktion in Abwickelrichtung, während das Aufwickeln von Testband - in dem gezeigten Beispiel bei Drehung im Uhrzeigersinn - über drei in Drehrichtung verteilte Mitnehmer 68 ermöglicht wird. Aufgrund der im Abstand befindlichen Sperrverzahnungen 26, 28 werden so störende Geräusche vermieden, und es treten keine unerwünschten Bremsmomente auf.

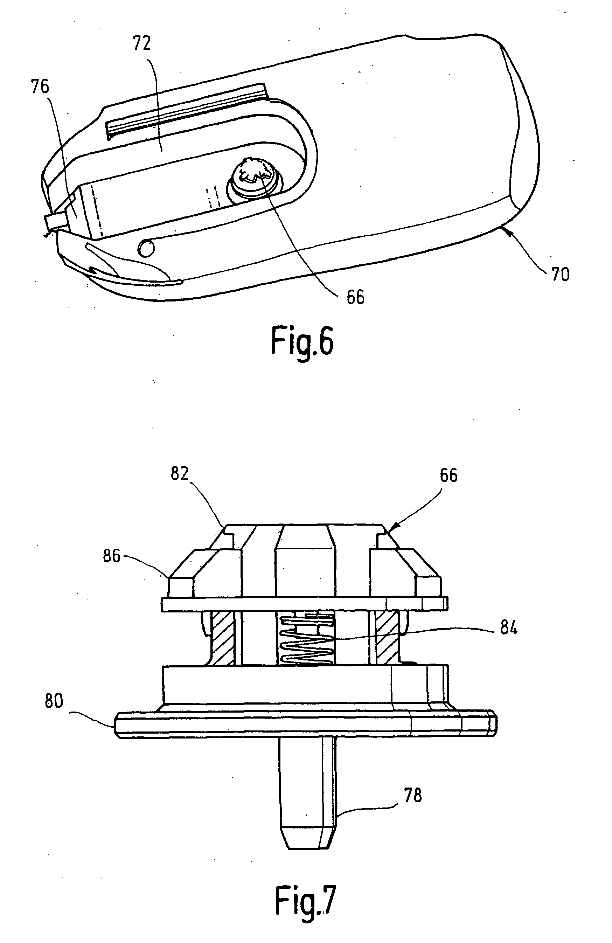

Fig. 6 zeigt ein Handgerät 70 mit einer Aufnahme 72, in die sich die Bandkassette 10 einsetzen lässt, um mit dem so gebildeten Messsystem einen weitgehend automatischen Messablauf zu ermöglichen. In der Aufnahme 72 wird die geräteseitige Messoptik 76 relativ zu der Applikationsspitze 20 positioniert. Hierbei erlaubt das vorstehend erläuterte Querspiel der Aufwickelspule 16 einen Ausgleich von Montage- und Positioniertoleranzen der eigens in dem Gerätegehäuse montierten Antriebsspindel 66.

Die in Fig. 7 gesondert gezeigte Antriebsspindel 66 besitzt einen die Aufnahme 72 bodenseitig durchsetzenden Abtriebszapfen 78, der über ein Zahnradgetriebe mit einem Antriebsmotor in dem Gerät 70 gekoppelt ist (nicht gezeigt). Auf dem Zapfen 78 sitzt eine Stützscheibe 80, auf welcher wiederum ein Kupplungskopf 82 über eine Schraubendruckfeder 84 bezüglich des Zapfens 78 axialbeweglich abgestützt ist. Beim Einsetzen der Kassette 10 in die Aufnahme 72 taucht der Kupplungskopf 82 in die Mitnahmebohrung 67 der Aufwickelspule 16 ein und kommt mit seinen Umfangsnocken 86 unter der Kraft der Feder 84 in Formschluss mit den Mitnehmern 68, so dass ein Drehmoment übertragen werden kann.

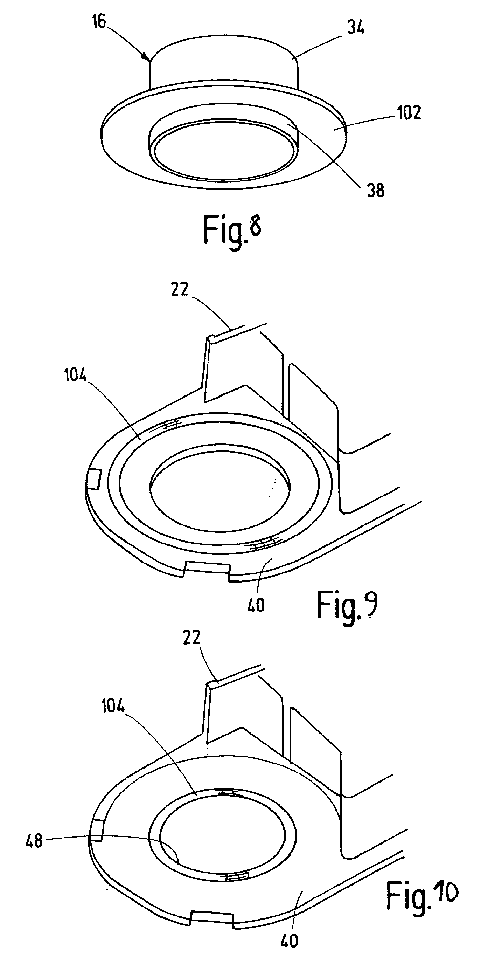

Bei der in Fig. 8 bis 10 gezeigten Ausführungsform ist anstelle der Sperrverzahnung 26, 28 eine von der Drehstellung und Drehrichtung unabhängige Reibschlusssperre vorgesehen. Gemäß Fig.8 bleibt hierbei der Bund 102 der Aufwickelspule 16 frei von einer Verzahnung. Für den Reibschluss ist an der Auflagefläche des Ringflansches 40 des Gehäuses 22 ein Reibring 104 angeformt, der entweder etwa in der Ringflächenmitte (Fig. 9) oder innen an dem die Flanschöffnung 48 begrenzenden Rand (Fig. 10) sitzt. Der Reibring 104 besteht aus einem thermoplastischen Elastomermaterial (TPE), das im selben Fertigungsschritt zusammen mit Dichtungskomponenten an das Gehäuse 22 im Spritzgießverfahren angespritzt wird. Auch hier wird der Reibschluss durch eine am Gehäuse 22 ausgebildete Rückstellfeder 56 entsprechend Fig. 4 unterstützt, während beim Eingriff der Antriebsspindel 66 entsprechend Fig. 5 die Aufwickelspule 16 entgegen der Federkraft angehoben und dabei der Reibschluss aufgehoben wird.

## Patentansprüche

1. Diagnostische Bandkassette, insbesondere für Blutzuckertests, mit einem analytischen Testband (12), einer Vorratsspule (14) zum Abspulen von unverbrauchtem Testband (12) sowie einer Aufwickelspule (16) zum Aufwickeln von verbrauchtem Testband (12), einem Gehäuse (22) für die Spulen (14,16) und einer Drehsicherung (24) für die Aufwickelspule (16) zumindest gegen ungewolltes Abwickeln von Testband (12),
**dadurch gekennzeichnet dass** die Drehsicherung (24) jeweils eine an gegeneinander weisenden Flächen (44,50) des Gehäuses (22) und der Aufwickelspule (16) angeordnete, durch eine Axialbewegung der Aufwickelspule (16) zwischen einer gegenseitigen Eingriffstellung und einer Freigabestellung schaltbare Sperrverzahnung (26,28) aufweist.

2. Bandkassette nach Anspruch 1, **dadurch gekennzeichnet, dass** die Sperrverzahnungen (26,28) bei geräteunabhängiger Handhabung der Bandkassette (10) in der Eingriffstellung stehen.

3. Bandkassette nach Anspruch 1 oder 2, **dadurch gekennzeichnet, dass** die Sperrverzahnungen (26,28) bei einem Geräteeinsatz der Bandkassette (10) vorzugsweise durch Einführen einer geräteseitigen Antriebsspindel (66) in die Aufwickelspule (16) aus der Eingriffstellung in die Freigabestellung bringbar sind.

4. Bandkassette nach einem der Ansprüche 1 bis 3, **dadurch gekennzeichnet, dass** die Aufwickelspule (16) in der Freigabestellung in beide Drehrichtungen frei drehbar ist, und dass in der Eingriffstellung beide Drehrichtungen der Aufwickelspule (16) gesperrt sind.

5. Bandkassette nach einem der Ansprüche 1 bis 4, **dadurch gekennzeichnet, dass** die Aufwickelspule (16) eine an einer Stirnseite verzahnte Zahnscheibe (32) als Sperrverzahnung (28) aufweist.

6. Bandkassette nach einem der Ansprüche 1 bis 5, **dadurch gekennzeichnet, dass** die Zahnscheibe (32) durch einen Bund eines mit dem Testband (12) umwickelbaren zylinderförmigen Spulenkörpers (34) gebildet ist, wobei die Sperrverzahnung (28) von dem Testband (12) abgewandt ist.

7. Bandkassette nach einem der Ansprüche 1 bis 6, **dadurch gekennzeichnet, dass** die Aufwickelspule (16) über eine Rückstellfeder (56) in Richtung der Spulenachse (30) an dem Gehäuse (22) abgestützt ist.

8. Bandkassette nach einem der Ansprüche 1 bis 7, **dadurch gekennzeichnet, dass** die Rückstellfeder (56) vorzugsweise als Blattfeder oder Spiralfeder an einer Wand (58) des Gehäuses (22) angeformt ist, und dass zwischen der Wand (58) und der Sperrverzahnung (26,28) ein Ringraum (64) für das Testband (12) freigehalten ist.

9. Bandkassette nach einem der Ansprüche 1 bis 8, **dadurch gekennzeichnet, dass** das Gehäuse (22) einen verzahnten Ringflansch (40) als Sperrverzahnung (26) aufweist.

10. Bandkassette nach einem der Ansprüche 1 bis 9, **dadurch gekennzeichnet, dass** die Aufwickelspule (16) in eine von einer körperlichen Drehachse freie Kammer (62) des Gehäuses (22) eingesetzt ist.

11. Diagnostische Bandkassette, insbesondere für Blutzuckertests, mit einem analytischen Testband (12), einer Vorratsspule (14) zum Abspulen von unverbrauchtem Testband (12) sowie einer Aufwickelspule (16) zum Aufwickeln von verbrauchtem Testband (12), einem Gehäuse (22) für die Spulen (14,16) und einer Drehsicherung (24) für die Aufwickelspule (16) zumindest gegen ungewolltes Abwickeln von Testband (12), **dadurch gekennzeichnet dass** die Drehsicherung (24) ein an einer Gehäusefläche (40) angebrachtes Reibelement (104) aufweist, welches bei einer Axialbewegung der Aufwickelspule (16) aus einer Freigabestellung in Reibschluss mit einer zugewandten Anlagefläche (50) der Aufwickelspule (16) gelangt.

12. Bandkassette nach Anspruch 11, **dadurch gekennzeichnet, dass** das Reibelement (104) durch einen koaxial zu der Aufwickelspule angeordneten Reibring gebildet ist.

13. Bandkassette nach Anspruch 11 oder 12, **dadurch gekennzeichnet, dass** das Reibelement (104) aus einem Elastomermaterial, vorzugsweise aus TPE besteht.

14. Bandkassette nach einem der Ansprüche 11 bis 13, **dadurch gekennzeichnet, dass** die Anlagefläche (50) durch einen Bund eines mit dem Testband (12) umwickelbaren zylinderförmigen Spulenkörpers (34) gebildet ist.

15. Bandkassette nach einem der Ansprüche 11 bis 14, **dadurch gekennzeichnet, dass** die Aufwickelspule (16) über eine Rückstellfeder (56) in Richtung der Spulenachse (30) an dem Gehäuse (22) abgestützt ist, so dass der Reibschluss unter der Rückstellkraft der Rückstellfeder erfolgt.

## Claims

1. Diagnostic tape cassette, particularly for blood sugar tests, comprising an analytical test tape (12), a supply spool (14) for winding off unused test tape (12) and a take-up spool (16) for winding used test tape (12), a housing (22) for the spools (14, 16) and a rotational lock (24) for the take-up spool (16) at least against inadvertent unwinding of test tape (12), **characterized in that** the rotational lock (24) has locking teeth (26, 28) disposed on both opposing faces (44, 50) of the housing (22) and the take-up spool (16), said teeth being shifted by an axial movement of the take-up spool (16) between a mutually engaged position and a release position.

2. Tape cassette according to claim 1, **characterized in that** the locking teeth (26, 28) are in the engaged position when the tape cassette (10) is handled independently of the device.

3. Tape cassette according to claim 1 or 2, **characterized in that** the locking teeth (26, 28) can be moved from the engaged position into the release position when the tape cassette (10) is used in a device preferably by inserting a drive spindle (66) of the device into the take-up spool (16).

4. Tape cassette according to one of the claims 1 to 3, **characterized in that** the take-up spool (16) can be freely rotated in both directions of rotation in the release position and both directions of rotation of the take-up spool (16) are locked in the engaged position.

5. Tape cassette according to one of the claims 1 to 4, **characterized in that** the take-up spool (16) has a toothed disk (32) with teeth on a front face as locking teeth (28).

6. Tape cassette according to one of the claims 1 to 5, **characterized in that** the toothed disk (32) is formed by a collar of a cylindrical spool body (34) around which the test tape (12) can be wound, wherein the locking teeth (28) faces away from the test tape (12).

7. Tape cassette according to one of the claims 1 to 6, **characterized in that** the take-up spool (16) is braced against the housing (22) in the direction of the spool axis (30) by means of a return spring (56).

8. Tape cassette according to one of the claims 1 to 7, **characterized in that** the return spring (56) is preferably formed as a leaf spring or spiral spring on a wall (58) of the housing (22) and that an annular space (64) is kept free for the test tape (12) between the wall (58) and the locking teeth (26, 28).

9. Tape cassette according to one of the claims 1 to 8, **characterized in that** the housing (22) has a toothed ring flange (40) as locking teeth (26).

10. Tape cassette according to one of the claims 1 to 9, **characterized in that** the take-up spool (16) is inserted into a chamber (62) of the housing (22) that is free of a physical axis of rotation.

11. Diagnostic tape cassette, particularly for blood sugar tests, comprising an analytical test tape (12), a supply spool (14) for winding off unused test tape (12) and a take-up spool (16) for winding used test tape (12), a housing (22) for the spools (14, 16) and a rotational lock (24) for the take-up spool (16) at least against inadvertent unwinding of test tape (12), **characterized in that** the rotational lock (24) has a friction element (104) attached to a housing surface (40) which is brought into frictional engagement with a facing contact surface (50) of the take-up spool (16) when the take-up spool (16) is moved axially out of a release position.

12. Tape cassette according to claim 11, **characterized in that** the friction element (104) is formed by a friction ring arranged coaxially with respect to the take-up spool.

13. Tape cassette according to claim 11 or 12, **characterized in that** the friction element (104) consists of an elastomer material, preferably TPE.

14. Tape cassette according to one of the claims 11 to 13, **characterized in that** the contact surface (50) is formed by a collar of a cylindrical spool body (34) around which the test tape (12) can be wound.

15. Tape cassette according to one of the claims 11 to 14, **characterized in that** the take-up spool (16) is braced against the housing (22) in the direction of the spool axis (30) by means of a return spring (56) such that the frictional engagement occurs under the restoring force of the return spring.

## Revendications

1. Cassette à bande diagnostique, en particulier pour tests de glycémie, avec une bande test analytique (12), une bobine de réserve (14) pour débobiner une bande test (12) non usagée et une bobine réceptrice (16) pour rembobiner une bande test (12) usagée, un boîtier (22) pour lesdites bobines (14, 16) et une protection anti-rotation (24) pour la bobine réceptrice (16) qui protège au moins contre le déroulement intempestif de bande test (12), **caractérisée en ce que** la protection anti-rotation (24) présente respectivement une denture de blocage (26, 28), disposée sur chacune des surfaces (44, 50) tournées l'une contre l'autre du boîtier (22) et de la bobine réceptrice (16), qui est commutable par un mouvement axial de la bobine réceptrice (16) entre une position d'engagement mutuel et une position de dégagement.

2. Cassette à bande selon la revendication 1, **caractérisée en ce que** les dentures de blocage (26, 28) se trouvent dans la position d'engagement lorsque la cassette à bande (10) est manipulée séparément de l'appareil.

3. Cassette à bande selon la revendication 1 ou 2, **caractérisée en ce que** les dentures de blocage (26, 28) peuvent être amenées de la position d'engagement à la position de dégagement, de préférence par introduction d'une tige d'entraînement (66) côté appareil dans la bobine réceptrice (16), lorsque la cassette à bande (10) est placée dans l'appareil.

4. Cassette à bande selon une des revendications 1 à 3, **caractérisée en ce qu'**en position de dégagement la bobine réceptrice (16) est susceptible de tourner librement dans les deux sens de rotation et qu'en position d'engagement les deux sens de rotation de la bobine réceptrice (16) sont bloqués.

5. Cassette à bande selon une des revendications 1 à 4, **caractérisée en ce que** la bobine réceptrice (16) présente un disque denté (32) sur une face frontale comme denture de blocage (28).

6. Cassette à bande selon une des revendications 1 à 5, **caractérisée en ce que** le disque denté (32) est formé par un épaulement d'un corps de bobine cylindrique (34) pouvant être enveloppé avec la bande test (12), la denture de blocage (28) étant détournée de la bande test (12).

7. Cassette à bande selon une des revendications 1 à 6, **caractérisée en ce que** la bobine réceptrice (16) s'appuie sur le boîtier (22) en direction de l'axe de bobine (30) par l'intermédiaire d'un ressort de rappel (56).

8. Cassette à bande selon une des revendications 1 à 7, **caractérisée en ce que** le ressort de rappel (56), de préférence sous forme de ressort à lames ou ressort en spirale, est formé sur une paroi (58) du boîtier (22) et qu'un espace annulaire (64) est réservé pour la bande test (12) entre ladite paroi (58) et la denture de blocage (26, 28).

9. Cassette à bande selon une des revendications 1 à 8, **caractérisée en ce que** le boîtier (22) présente une bride annulaire dentée (40) comme denture de blocage (26).

10. Cassette à bande selon une des revendications 1 à 9, **caractérisée en ce que** la bobine réceptrice (16) est placée dans une chambre (62) du boîtier (22), exempte d'axe de rotation physique.

11. Cassette à bande diagnostique, en particulier pour tests de glycémie, avec une bande test analytique (12), une bobine de réserve (14) pour débobiner une bande test (12) non usagée et une bobine réceptrice (16) pour rembobiner une bande test (12) usagée, un boîtier (22) pour lesdites bobines (14, 16) et une protection anti-rotation (24) pour la bobine réceptrice (16), protégeant au moins contre le déroulement intempestif d'une bande test (12), **caractérisée en ce que** la protection anti-rotation (24) présente un élément de friction (104) monté sur une surface de boîtier (40) et qui, lors d'un mouvement axial de la bobine réceptrice (16), passe d'une position de dégagement à une liaison de friction avec une surface d'appui (50) en regard de la bobine réceptrice (16).

12. Cassette à bande selon la revendication 11, **caractérisée en ce que** l'élément de friction (104) est formé par un anneau de friction, disposé coaxialement à la bobine réceptrice.

13. Cassette à bande selon la revendication 11 ou 12, **caractérisée en ce que** l'élément de friction (104) est constitué d'un matériau elastomère, de préférence de TPE.

14. Cassette à bande selon une des revendications 11 à 13, **caractérisée en ce que** la surface d'appui (50) est formée par un épaulement d'un corps de bobine cylindrique (34) pouvant être enveloppé avec la bande test (12).

15. Cassette à bande selon une des revendications 11 à 14, **caractérisée en ce que** la bobine réceptrice (16) s'appuie sur le boîtier (22) en direction de l'axe de bobine (30) par l'intermédiaire d'un ressort de rappel (56), de telle sorte que la liaison de friction a lieu sous la force de rappel du ressort de rappel.
